# DEMANDE DE BREVET EUROPEEN

(11) **EP 1 336 396 A1**
(43) Date de publication de la demande: **20.08.2003**
(21) Numéro de dépôt: 03356024.4
(22) Date de dépôt: 14.02.2003
(51) Int. Cl.: A61F 2/40

(54) **Composant glénoidien de prothèse d'épaule et prothèse totale d'épaule incorporant un tel composant**

(30) Priorité: 15.02.2002 FR 0201944
(71) Demandeur: TORNIER SA, 38330 Saint Ismier (FR)
(72) Inventeur: Tornier, Alain, 38330 Saint Ismier (FR)
(74) Mandataire: Myon, Gérard

(57) **Abrégé**

Ce composant comprend un socle (21), apte à être immobilisé sur la glène d'une épaule, et un élément (24) formant une surface d'articulation convexe (S₂) apte à coopérer avec un composant huméral de prothèse. Cet élément (24) est apte à être monté sur le socle (21) dans plusieurs positions correspondant respectivement à différentes positions (P₁-P₃) de la surface d'articulation (S₂) par rapport à la glène.

## Description

L'invention a trait à un composant glénoïdien de prothèse d'épaule et à une prothèse totale d'épaule comprenant un tel composant.

Dans le domaine des prothèses d'épaule, il est connu, par exemple de US-A-3,978,528, de constituer une prothèse inversée dans laquelle une surface articulaire convexe est solidaire de la glène, alors qu'une surface articulaire concave est solidaire de l'humérus, la coopération de ces surfaces permettant de recréer une articulation au niveau de l'épaule. Dans ce genre de prothèses, on peut utiliser un socle fixé sur la glène et sur lequel est monté un élément formant la surface d'articulation convexe.

La conception des prothèses inversées du type mentionné ci-dessus répond à la nécessité de restaurer l'indolence et la fonction de l'articulation de l'épaule dans les cas de destruction massive de la coiffe des éléments assurant la rotation. Dans ce cas, l'élévation du bras est assurée uniquement par le muscle deltoïde. Il importe donc que les bras de levier correspondant aux points d'application des forces subies par l'articulation soient déterminés avec une grande précision, afin de limiter, autant que faire se peut, les efforts que doit développer un patient pour soulever un bras équipé d'une prothèse d'épaule. Il arrive en effet que les prothèses connues induisent une gène pour les patients.

C'est à ces inconvénients qu'entend plus particulièrement remédier l'invention en proposant un composant glénoïdien de prothèse d'épaule qui permet de tirer le meilleur parti de la tension musculaire, en réduisant les efforts nécessaires à l'élévation du bras.

Dans cet esprit, l'invention concerne un composant glénoïdien de prothèse d'épaule qui comprend un socle, apte à être immobilisé sur la glène d'une épaule, et un élément formant une surface d'articulation convexe apte à coopérer avec un composant huméral de prothèse, cet élément étant prévu pour être monté sur ce socle. Ce composant est caractérisé en ce que l'élément formant la surface convexe est apte à être monté sur le socle dans plusieurs positions correspondant respectivement à différentes positions de la surface d'articulation convexe par rapport à la glène.

Grâce à l'invention, on peut utiliser les différentes positions possibles de la surface d'articulation convexe par rapport à la glène pour faire varier, en phase per-opératoire, la position de cette surface, ce qui permet d'ajuster avec précision la géométrie de l'articulation ainsi recréée.

Selon des aspects avantageux mais non obligatoires de l'invention, ce composant incorpore une ou plusieurs des caractéristiques suivantes :
- L'élément formant une surface d'articulation est constitué d'une pièce en tronçon de sphère formant la surface d'articulation et d'une platine apte à supporter cette pièce et pourvue de moyens de positionnement et de fixation par rapport au socle.
- Il est prévu des moyens d'indexation de la position de l'élément formant la surface d'articulation convexe par rapport au socle. Ces moyens d'indexation, c'est-à-dire de positionnement incrémental, peuvent comprendre une extension faisant saillie à partir de l'élément précité, ainsi que plusieurs logements prévus sur le socle pour la réception sélective de cette extension. Selon le logement dans lequel est reçue l'extension précitée, on obtient différentes positions de la surface d'articulation convexe par rapport à la glène. Les logements précités sont avantageusement délimités par les bords d'une encoche ménagée dans un voile du socle destiné à être plaqué contre la glène. On peut, en outre, prévoir que les moyens d'indexation comprennent des orifices de passage de vis cinématiquement liées à l'élément formant la surface d'articulation convexe, ces orifices étant répartis au voisinage des logements précités pour recevoir ces vis, en fonction de la position de l'extension dans l'un de ces logements.
- Lorsque l'élément formant l'extension est bipartite comme indiquée ci-dessus, l'extension qu'il comprend peut être creuse et former un passage pour une vis de fixation de la pièce en tronçon de sphère sur la platine.
- Les différentes positions de l'élément formant la surface d'articulation convexe par rapport au sol sont au nombre de trois et sont décalées, dans le plan sagittal, d'environ 5 mm les unes par rapport aux autres.
- Le socle est pourvu d'une aile d'appui acromio-coracoïdien qui s'étend globalement selon une direction perpendiculaire à un voile du socle dans lequel sont prévus des moyens de montage de l'élément formant la surface d'articulation convexe.

L'invention concerne également une prothèse totale d'épaule qui comprend un composant glénoïdien tel que précédemment décrit. Une telle prothèse est plus confortable pour le patient que les prothèses inversées connues.

L'invention sera mieux comprise et d'autres avantages de celle-ci apparaîtront plus clairement à la lumière de la description qui va suivre d'un composant glénoïdien et d'une prothèse conformes à son principe, donnée uniquement à titre d'exemple et faite en référence aux dessins annexés dans lesquels :
- La figure 1 est une représentation schématique de principe d'une prothèse conforme à l'invention implantée sur un patient ;
- La figure 2 est une vue de face, dans le sens de la flèche F₁ à la figure 1, du composant glénoïdien de la prothèse de la figure 1 ;
- La figure 3 est une vue de côté du composant de la figure 2 ;
- La figure 4 est une vue de face d'un socle appartenant au composant des figures 2 et 3 ;
- La figure 5 est une vue de face d'une platine appartenant au composant des figures 2 et 3 et

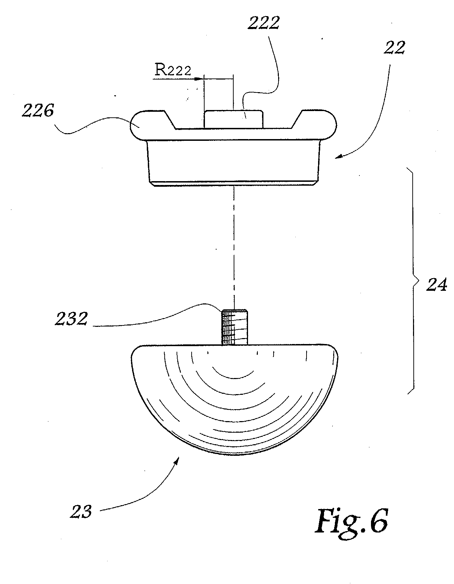

- La figure 6 est une vue éclatée d'un élément formant une surface d'articulation convexe, comprenant la platine de la figure 5 et destiné à coopérer avec le socle de la figure 4 pour constituer le composant des figures 2 et 3.

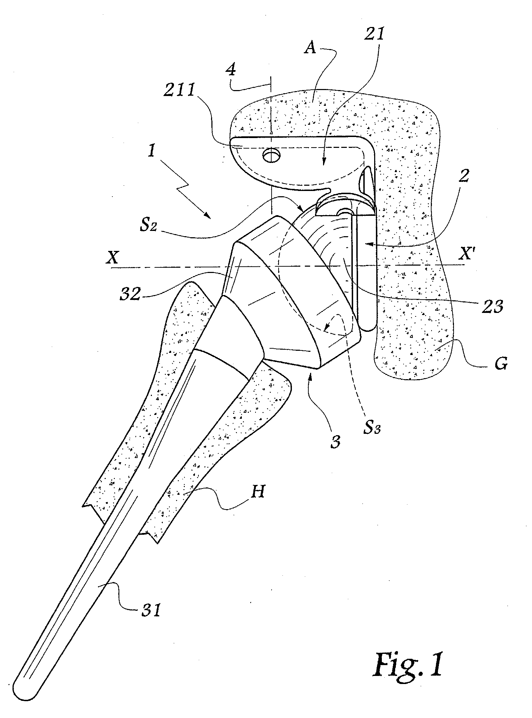

La prothèse 1 représentée à la figure 1 comprend un premier composant 2 fixé sur la glène G d'une épaule ainsi qu'un second composant 3 fixé dans l'humérus H correspondant.

Le composant 3 comprend une tige 31 destinée à être ancrée dans le canal médullaire de l'humérus H. Le composant 3 comprend également une partie 32 définissant une surface concave S₃ en forme de tronçon de sphère.

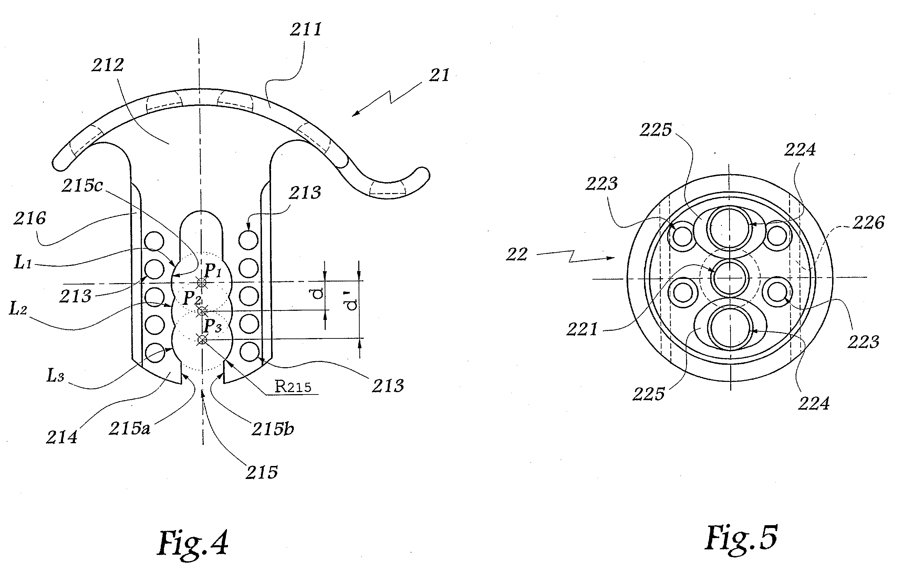

Le composant 2 comprend un socle 21 prévu pour être immobilisé sur l'acromion A grâce à des vis 4 traversant une aile 211 du socle 21 destiné à venir en appui contre l'acromion. Cette aile 211 est de forme courbe et s'étend globalement perpendiculairement à un voile 212 du socle 21 plus particulièrement visible à la figure 4. Aux figures 1 à 3, les traces des vis 4 ont été représentées par des traits d'axe pour la clarté du dessin.

L'aile 211 est facultative et le socle 21 peut être fixé sur la glène par tout moyen approprié.

Le composant 2 comprend également une platine métallique 22 destinée à être associée à une pièce métallique 23 dont la surface extérieure 231 constitue la surface S₂ précédemment mentionnée.

L'assemblage entre la platine 22 et la pièce 23 est effectué grâce à une vis 232 solidaire de la pièce 23 et propre à s'engager dans un orifice 221 ménagé dans la partie centrale de la platine 22, à l'intérieur d'une extension cylindrique à base circulaire 222 qui est creuse pour constituer l'orifice 221. L'orifice 221 est taraudé sur une partie au moins de sa longueur afin de coopérer avec la vis 232.

Le voile 212 est pourvu de plusieurs perçages 213 ménagés dans deux ailes 214 formées de part et d'autre d'une encoche 215 ménagée dans le voile 212. Les bords longitudinaux 215a et 215b de cette encoche forment chacun trois secteurs d'arc de cercle 215c centrés sur trois points imaginaires P₁, P₂ et P₃ et dont le rayon R₂₁₅ est sensiblement égal au rayon R₂₂₂ de l'extension 222.

Ainsi, la platine 22 peut être plaquée contre le voile 212 du socle 21 dans trois positions correspondant respectivement à l'engagement de l'extension 222 dans chacun des trois logements L₁, L₂, L₃ formés par l'encoche 215 autour de chacun des points P₁, P₂ et P₃.

Le logement L₁ est tel que, si l'extension 222 est engagée dans ce logement, le centre géométrique C₂ de la surface S₂ est sensiblement aligné avec un axe médian X-X' passant par le centre géométrique de la pyramide glenoïdienne dans le plan frontal.

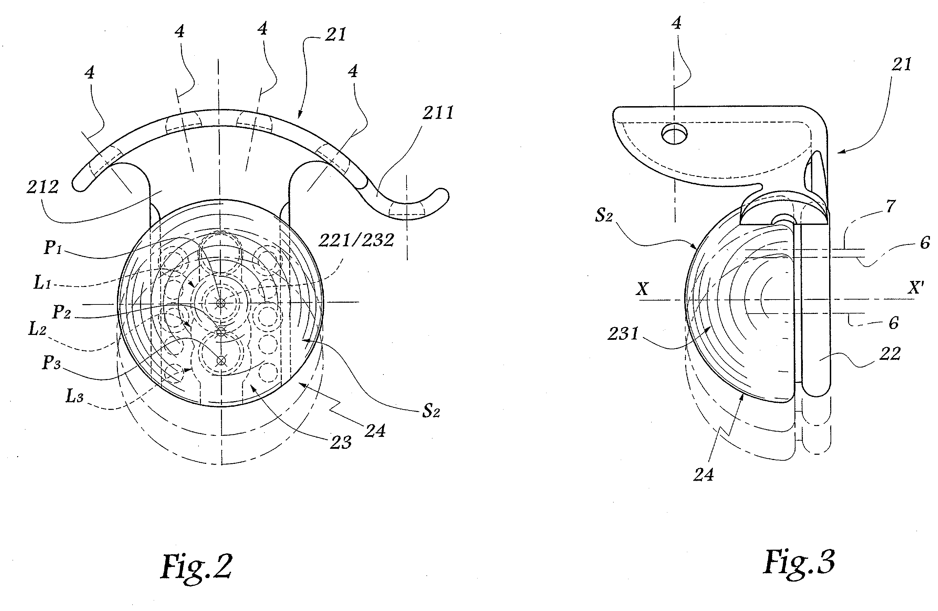

La distance d entre les points P₁ et P₂ est égale à environ 5 mm, de même que la distance d' entre les points P₂ et P₃. Il est donc possible d'obtenir, à partir de la position de la surface S₂ représentée en traits pleins aux figures 2 et 3, position qui correspond à l'introduction de l'extension 222 dans le logement L₁, deux positions d'excentration verticale vers le bas dans le plan sagittal qui correspondent respectivement à des déplacements de 5 et 10 mm, dans le plan sagittal et vers le bas. Ces seconde et troisième positions, qui correspondent respectivement au positionnement de l'extension 222 dans le logement L₂ et dans le logement L₃, sont représentées en traits mixtes aux figures 2 et 3.

Lorsque la position de la platine 22 par rapport au socle 21 a été déterminée, cette position est fixée par l'introduction, dans quatre orifices 223 prévus sur la platine 22, de vis 6 destinées à pénétrer dans certains des perçages 213. Les vis en question sont représentées par leurs traits d'axe 6 à la figure 3.

Les perçages 213 peuvent être taraudés, auquel cas les vis 6 viennent en prise dans ces perçages. Les perçages 213 peuvent également être lisses, auquel cas les vis 6 sont directement vissées dans la glène.

Il est par ailleurs prévu dans la platine 22 deux orifices 224 disposés de part et d'autre de l'orifice 221 et prévus pour le passage de vis de fixation de la platine 22 par rapport à la glène G. Ces vis sont représentées par leur trait d'axe 7 aux figures 2 et 3 et elles ont des diamètres supérieurs aux vis 6. Les vis 7 pénètrent dans la glène G en traversant l'encoche 215.

La platine 22 forme, autour des orifices 224, des sièges 225 de forme arrondie et concave adaptés pour coopérer avec les têtes des vis 7, de telle sorte que les vis 7 peuvent avoir différentes orientations par rapport au voile 212, ce qui permet de sélectionner au choix la partie de la glène G dans laquelle sont implantées les vis 7.

La platine 22 est pourvue de deux retours 226 prévus pour venir en recouvrement des bords longitudinaux 216 du voile 212.

Grâce aux vis 5 à 7, il est donc possible d'immobiliser la surface S₂ par rapport au socle 21 dans l'une des trois configurations représentées respectivement en traits pleins et en traits mixtes aux figures 2 et 3.

Les trois positions possibles de l'élément 24, formé de la platine 22 et de la pièce 23, par rapport au socle 21 correspondent à la superposition du centre de l'extension 22 avec l'un des points P₁, P₂ ou P₃.

La mise en place du composant 2 s'effectue en positionnant en premier lieu le socle 21 et en le fixant sur l'acromion grâce aux vis 4. Ensuite, grâce à des éléments fantômes qui reproduisent la forme de la surface S₂, le chirurgien peut effectuer des essais de positions et choisir la position la plus adaptée pour l'élément formé de la platine 22 et de la pièce 23. Une fois cette position choisie, la platine 22 est solidarisée au socle 21 et l'ensemble est fixé à la glène G par les vis 7. La pièce 23 en partie de sphère est ensuite impactée sur la platine et immobilisée sur celle-ci grâce à la vis 5.

Selon une variante non représentée de l'invention, le réglage de la position de la surface S₂ par rapport au socle 21 peut également avoir lieu selon une direction antéro-postérieure, c'est-à-dire vers la gauche ou vers la droite à la figure 2. Dans ce cas, la géométrie des logements prévus sur le socle 21 est adaptée.

## Revendications

1. Composant glénoïdien (2) de prothèse d'épaule (1) comprenant un socle (21), apte à être immobilisé sur la glène (G) d'une épaule, et un élément (24) formant une surface d'articulation convexe (S₂) apte à coopérer avec un composant huméral (3) de prothèse, ledit élément étant prévu pour être monté sur ledit socle, **caractérisé en ce que** ledit élément (24) est apte à être monté sur ledit socle (21) dans plusieurs positions (P₁-P₃) correspondant respectivement à différentes positions de ladite surface d'articulation convexe (S₂) par rapport à la glène (G).

2. Composant selon la revendication 1, **caractérisé en ce que** ledit élément (24) formant une surface d'articulation est constitué d'une pièce (23) en tronçon de sphère formant ladite surface d'articulation (S₂) et d'une platine (22) apte à supporter ladite pièce et pourvue de moyens (6, 22) de positionnement et de fixation par rapport audit socle (21)

3. Composant selon l'une des revendications précédente, **caractérisé en ce qu'**il comprend des moyens (6, 22, 213, 215c, 223, L₁-L₃) d'indexation de la position dudit élément (24) par rapport audit socle (21).

4. Composant selon la revendication 3, **caractérisé en ce que** lesdits moyens d'indexation comprennent une extension (222) faisant saillie à partie dudit élément (24) et plusieurs logements (L₁-L₃) prévus sur ledit socle (21) pour la réception de ladite extension.

5. Composant selon la revendication 4, **caractérisé en ce que** lesdits logements (L₁-L₃) sont délimités par les bords (215a, 215b, 215c) d'une encoche (215) ménagée dans un voile (212) dudit socle destiné à être plaqué contre la glène (G) .

6. Composant selon l'une des revendications 4 ou 5, **caractérisé en ce que** lesdits moyens d'indexation comprennent des orifices (213) de passage de vis (6) cinématiquement liées audit élément (24), lesdits orifices étant répartis au voisinage desdits logements (L₁-L₃) pour recevoir lesdites vis en fonction de la position de ladite extension (222) dans l'un desdits logements.

7. Composant selon la revendication 2 et l'une des revendications 4 à 6, **caractérisé en ce que** ladite extension (222) est creuse et forme un passage (221) pour une vis (5) de fixation de ladite pièce (23) sur ladite platine (22).

8. Composant selon l'une des revendications précédentes, **caractérisé en ce que** lesdites positions (P₁-P₃) sont au nombre de trois et sont décalées (d, d') dans le plan sagittal, d'environ 5 mm les unes par rapport aux autres.

9. Composant selon l'une des revendications précédentes, **caractérisé en ce que** ledit socle (21) est pourvu d'une aile (211) d'appui acromio-coracoïdien s'étendant globalement selon une direction perpendiculaire à un voile (212) dudit socle dans lequel sont prévus des moyens (213, 215, L₁-L₃) de montage dudit élément (24) formant surface d'articulation convexe (S₂).

10. Prothèse totale d'épaule (1), **caractérisée en ce qu'**elle comprend un composant glénoïdien (2) selon l'une des revendications précédentes.
